# EUROPEAN PATENT SPECIFICATION

(11) **EP 3 024 754 B1**
(45) Date of publication and mention of the grant of the patent: **28.03.2018**
(21) Application number: 14744499.6
(22) Date of filing: 17.07.2014
(51) Int. Cl.: B65D 83/04, A61M 5/00, A61M 5/32

(54) **NEEDLE STORAGE DEVICE**
NADELAUFBEWAHRUNGSVORRICHTUNG
DISPOSITIF DE STOCKAGE D'AIGUILLE

(30) Priority: 22.07.2013 EP 13177490
(43) Date of publication of application: 01.06.2016
(73) Proprietor: Sanofi-Aventis Deutschland GmbH, 65929 Frankfurt am Main (DE)
(72) Inventor: DASBACH, Uwe, 65926 Frankfurt am Main (DE); NOBER, Peter, 54597 Rommersheim (DE); BAUMEYER, Martin, 04328 Leipzig (DE)
(74) Representative: Finger, Catrin
(86) International application number: PCT/EP2014/065420
(87) International publication number: WO 2015/011022

(56) References cited:
- EP-A1- 2 522 379
- EP-A1- 2 537 544
- WO-A1-02/11798
- GB-A- 2 459 772

## Description

### Technical Field

The invention relates to a needle storage device comprising a plurality of needle storage compartments for used and/or unused needles of a drug delivery device.

### Background of the Invention

Patients suffering from diseases like diabetes have to frequently self-administer injections. Drug delivery devices like auto-injectors or pen injectors have been developed to facilitate self-administering injections. Typically, such drug delivery devices are re-usable and refitted with sterile injection needle assemblies to minimize the risk of infections.

Portable needle storage devices like needle magazines or needle dispensers contain a plurality of such sterile injection needle assemblies that are adapted to be mounted to the drug delivery devices. The needle storage devices supplement the drug delivery devices to facilitate safe self-administration of the medicament. Additionally, the needle storage device may be used as a disposal container for used injection needles to reduce the risk of accidental needle stick injuries caused by contaminated injection needles.

GB 2 459 772 A discloses a rotary dispenser comprising a hollow shell housing a rotary internal carousel with an array of radial contents storage compartments for selective presentation to an access port in a shell wall. Then carousel is configured as an open sided drum or tray bounded by a circumferential wall punctuated by a series of access ports for each radial compartments. The rotary dispenser has a rotary index mechanism which acts as an indicator and is placed between an outer housing and the inner carousel and may have an internal segmented track interactive with a detent on a rotary carousel. The dispenser mechanism may be rotated using an axial push down button or a rotating control knob. The rotation of the carousel is controlled by a ratchet mechanism having a dog on the outer face of the push down button, the dog interacting with a sequence of ratchet slots on the central column of the carousel. Preferably the rotary carousel has an ovoid shell and is used as an injection needle or needle cartridge dispenser and can receive used needles for safe disposal.

EP 2 537 544 A1 discloses a needle assembly storage device comprising an array of needle assembly storage compartments and a slider movably disposed on the array. Each of the needle assembly storage compartments includes an opening, rails disposed on opposing sides of the opening, and a latch hingedly connected to the needle assembly storage compartment. The latch has an open position and a closed position at least partially covering the opening. The slider includes an opening adapted to receive an injection device, arms adapted to engage the rails and a trailer. Movement of the slider relative to the array in a first direction causes the trailer to move the latch in the open position to the closed position.

EP 2 522 379 A1 discloses a needle assembly storage device comprising a case adapted to contain an array of needle assembly storage compartments and a band rotatably disposed on the case, the band including an opening. Rotation of the band aligns the opening with an opening of a given one of the needle assembly storage compartments.

WO 02/11798 A1 discloses a needle magazine for storing a plurality of needle assemblies and for selectively dispensing said needle assemblies there from. The needle magazine comprises a cylinder-shaped base member with an upper surface and a bottom surface. These two surfaces are parallel to each other and connected by a cylinder-shaped surface. The base-member is provided with a number of compartments each having the form of a sector of a circle. Each compartment contains a needle assembly positioned in a horizontal position. The base member is at least partly covered by a cover having a first part parallel with the upper surface and a second part parallel with the cylinder-shaped surface of the base member, and which second part is provided with an opening through which access to each compartment can be gained radial of the axis of rotation of the cover.

### Summary of the Invention

It is an object of the present invention to provide a needle storage device with an improved, in particular safe storage of used needles in the needle storage device.

The object is achieved by a needle storage device according to claim 1.

Exemplary embodiments of the invention are given in the dependent claims. The needle storage device according to the present invention comprises a housing having an opening adapted to receive a needle of a drug delivery device, needle storage compartments moveably disposed within the housing, and an indicator pivotably disposed within the housing and adapted to activate an acoustic and/or tactile feedback when a needle of a drug delivery device is stored in one of the needle storage compartments which is aligned with the opening. The indicator comprises a base plate pivotably mounted to the housing about a pivotal axis. In the area of the opening a fin is provided and adapted to serve as a stop for the base plate in an initial state.

In an exemplary embodiment, the needle storage device further comprises a spring is arranged between the indicator and the housing. The spring is arranged on the pivotal axis of the indicator. The spring is adapted to be tensioned between the indicator and the housing during insertion of the drug delivery device by pivoting of the indicator with respect to the housing. The spring is formed as a leg spring. The base plate and the housing are moveable relative to each other by a slot-pin guidance. The needle storage device further comprises an indicator arm is provided which is flexible and guided on a crank formed by an inner surface of the housing. The crank comprises at least one ramp on which the indicator arm is guided and prestressed during insertion of the drug delivery device with its needle pointing into the opening as well as into the aligned needle storage compartment. After storage of the needle and removal of the drug delivery device without the needle the indicator arm slides over the ramp of the crank and activates the acoustic and/or tactile feedback.

In an exemplary embodiment, the housing comprises an outer shape and/or sections with a substantially circular, semicircular, oval or elliptic shape.

In an exemplary embodiment, the needle storage device further comprises a door provided for allowing access to the opening.

In an exemplary embodiment, the needle storage device further comprises a drive and conveyor chain adapted to deliver the needle storage compartments within the housing such that at least one of the needle storage compartments is aligned with the opening. The invention allows that the user knows when the drug delivery device can be removed from the needle storage device. Due to the acoustic and/or tactile feedback of the indicator integrated into the needle storage device, the user knows if the needle of the drug delivery device is detached in the needle storage compartment which is aligned with the opening of the needle storage device. Furthermore, in a case of a needle storage device with a chain of needle storage compartments it is not possible to insert the needle back again into the needle storage compartment after removing the needle because an automatic mechanism is provided that allows to move "used" needle storage compartments.

Further scope of applicability of the present invention will become apparent from the detailed description given hereinafter. However, it should be understood that the detailed description and specific examples, while indicating exemplary embodiments of the invention, are given by way of illustration only, since various changes and modifications within the scope of the invention as defined in the appended claims, will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows a perspective view of an exemplary embodiment of a needle storage device with an integrated indicator,
- Figure 2: shows a perspective view of another exemplary embodiment of a needle storage device with an integrated indicator,
- Figure 3: shows a perspective view of a further exemplary embodiment of a needle storage device with an integrated indicator, and
- Figure 4: shows a perspective view of an exemplary embodiment of an indicator disposed within a housing of a needle storage device.

Corresponding parts are marked with the same reference symbols in all figures.

### Detailed Description

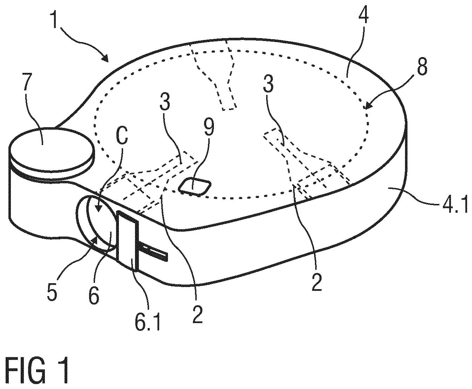

**Figure 1** shows an isometric view of an exemplary embodiment of a needle storage device 1 arranged to contain a plurality of needle storage compartments 2. The needle storage device 1 provides a sterile and safe transport receptacle for the plurality of unused or used needles 3 of a drug delivery device (not shown) retained in the needle storage compartments 2.

The needle storage device 1 comprises a substantially cylindrical housing 4 with a low profile and having a lateral wall 4.1 that has a section of substantially semi-circular shape. An opening 5 is formed into the lateral wall 4.1 that provides access to one of the needle storage compartments 2 aligned with the opening 5.

A door 6 arranged as a slider may be positioned in an open position (not shown) and in a closed position C to respectively provide and block access to the opening 5. The door 6 comprises a door handle 6.1 that is manually slid to move the door 6 between the open position and the closed position C.

The needle storage device 1 is used as a container for the needles 3 adapted to be mounted to a distal end of a drug delivery device, e.g. an auto-injector or a pen injector. The distal end of the drug delivery device is inserted into the opening 5 to attach the needle 3 and/or a needle assembly comprising a needle hub with a needle to the drug delivery device. In particular, a needle hub (not shown) affixed to the needle 3 is adapted to be mounted to the distal end of the drug delivery device upon insertion thereof into the opening 5 of the needle storage device 1.

The needle 3 retained in the needle storage compartment 2 may in particular be arranged as a double pointed needle having a pointed proximal tip. The drug delivery device may contain a medical ampoule containing a liquid medicament. Upon mounting of the needle 3 to the drug delivery device, the proximal tip of the needle 3 pierces a septum sealing the medical ampoule to establish fluid communication.

The needle storage device 1 comprises additionally an actuation element 7 that is adapted to be manually actuated to activate a drive and conveyor chain 8 for conveying the needle storage compartments 2 within the housing 4 in a manner that one of the needle storage compartments 2 is accessible through the opening 5. The needle storage device 1 acts both as a dispenser for sterile unused injection needles 3 and as a disposal device for used injection needles 3. After an injection has been performed, the used injection needle 3 is reinserted into the needle storage compartment 2 for safe disposal.

The actuation element 7 is arranged as a dial wheel that may be turned to activate the drive and conveyor chain 8 so as to deliver the needle storage compartments 2 within the housing 4. Manual activation of the actuation element 7 causes the drive and conveyor chain 8 to forward the needle storage compartments 2, e.g. disposed in a chain, by a defined step distance so that one of needle storage compartments 2 is properly aligned with the opening 5 disposed in the housing 4.

Alternatively, the actuation element 7 may be designed as a roller rotatably mounted with respect to the housing 4. The roller may cover a substantial height of the lateral wall 4.1 of the housing 4.

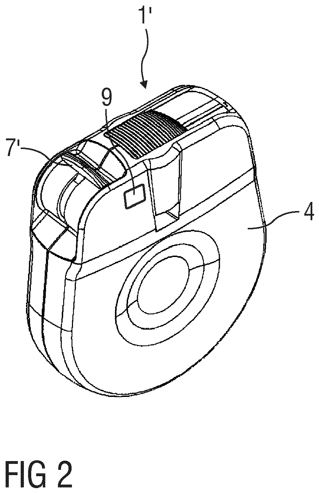

**Figure 2** shows an isometric view of an alternative embodiment of a needle storage device 1'. An actuation element 7' is arranged as a spring loaded lever that pivots about an angle of approximately 90 degrees upon manual actuation to activate the integrated drive and conveyor chain 8 forwarding the needle storage compartments 2 retained within the housing 4. After manual actuation, the lever is returned to the position shown in figure 2 by a spring. Thus, the design of the actuation element 7' only allows for a manual actuation in one rotational direction so as to forward the needle storage compartments 2 in the first direction.

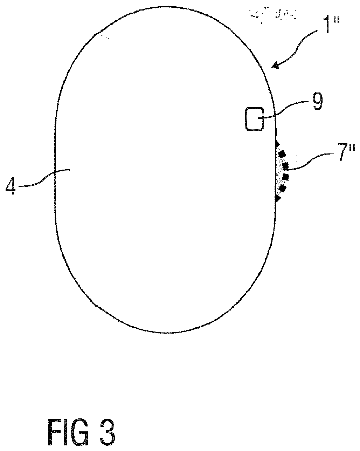

**Figure 3** shows a top view of another alternative embodiment of the needle storage device 1" comprising the housing 4 having a substantially elliptic cross-section. The actuation element 7" is arranged as rotatable dial wheel.

The housing 4 may comprise different outer shapes and storage sections with substantially circular, semicircular, oval or elliptic outer shapes and storage sections.

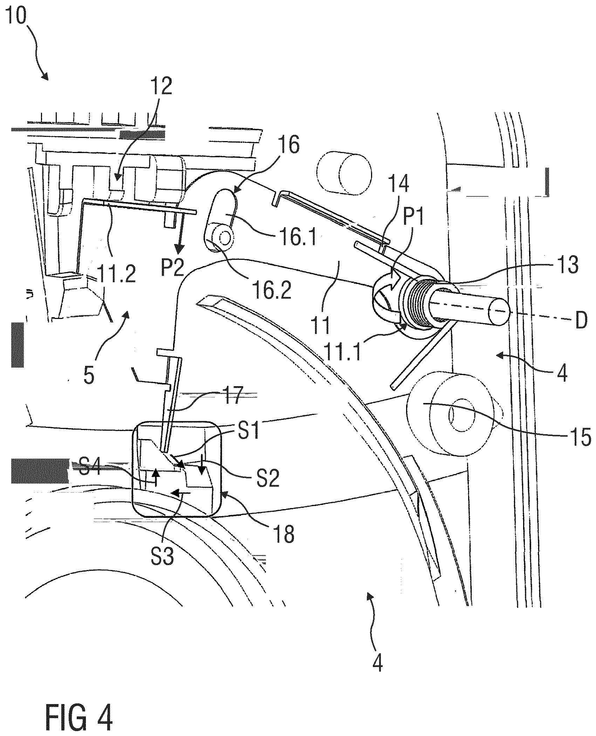

Each needle storage device 1, 1' and 1" comprises a touch element 9 capable of indicating if a needle 3 which is inserted into the needle storage compartment 2 aligned with the opening 5 is safely fixed and thus stored in that needle storage compartment 2. The touch element 9 is for example coloured or its surface is structured so that a user knows where he has to put his hands to feel a tactile feedback from an integrated indicator 10 which is shown in figure 4 in more detail.

**Figure 4** shows an exemplary embodiment of an indicator 10 integrated and disposed within a housing 4 of a needle storage device 1, 1' or 1".

The indicator 10 is pivotably disposed within the housing 4 and adapted to activate an acoustic and/or tactile feedback when a needle 3 of a drug delivery device is stored in one of the needle storage compartments 2 which is aligned with the opening 5. The indicator 10 comprises a base plate 11 pivotably mounted to the housing 4 about a pivotal axis D. The base plate 11 is formed as a profile, e.g. as an L-profile or a bent arm, and made of or comprising plastic or metal. About the pivotal axis D, the base plate 11 comprises an enlarged surface 11.1 with a hole through which the pivotal axis D is arranged.

The base plate 11 is pivotably movable as indicated by arrow P1 with respect to the housing 4 between an initial position shown in figure 4 in which no drug delivery device is inserted in the opening 5 and a storage position (not shown) in which a drug delivery device is inserted in the opening 5.

For an automatic reset of the base plate 11 from the storage position into the initial position after the drug delivery device is removed from the opening 5, a spring 13 is arranged between the indicator 10, in particular a first stop 14 on the indicator 10, and a second stop 15 on the housing 4.

In a possible embodiment, the spring 13 is formed as a leg spring and arranged on the pivotal axis D of the indicator 10 as it is shown in figure 4.

To support the movement of the indicator 10 relative to the housing 4 between the initial position and the storage position, a slot-pin guidance 16 is provided. In the shown embodiment, the indicator 10 comprises a slot 16.1 and a corresponding pin 16.2 protrudes from the inner surface of the housing 4. In a not shown embodiment, the housing may comprise a slot and the indicator may comprise a corresponding pin. Further, in the area of the opening 5 of the needle storage device 1 a fin 12 is provided and adapted to serve as an end stop for the base plate 11, in particular of an edge 11.2 of the base plate 11 in the initial state in which no drug delivery device is inserted in the opening 5. An end of the fin 12 towards the edge 11.2 comprises a curved contour or is formed as a hook.

Furthermore, the indicator 10 comprises an indicator arm 17 and a crank 18 on which the indicator arm 17 is guided during the movement of the indicator 10 between its initial and storage positions.

During operation of the needle storage device 1, e.g. during removal and attachment of an unused needle 3 to a drug delivery device or during detachment and storage of a used needle 3 into the drug delivery device, the drug delivery device is inserted into the opening 5 causing that the fin 12 pushes and moves the indicator 10, in particular the base plate 11 from the initial position (shown in figure 4) into the storage position (not shown indicated by arrow P2 and using the curved contour of the fin 12.

In particular, the indicator 10 is pivotably moved about the pivotal axis D. Due to the movement of the indicator 10, the spring 13 is adapted to be tensioned between the indicator 10 and the housing 4, in particular between the first and second stops 14 and 15. Further, the indicator arm 17, in particular at least its flexible end is guided at least on a ramp of the crank 18 such that the indicator arm 17 is pre-stressed. The indicator arm 17 is guided over the crank 18, in particular a structural surface with ramps and edges, in a given way according to guiding arrows S1 to S4 during the whole movement of the drug delivery device into and out off the opening 5 of the needle storage device 1. The crank 18 may have different shapes with different ramps and edges. In an example, the crank 18 is formed by an inner surface of the housing 4.

At the highest level or height of the crank 18 and for example when the drug delivery device with its needle 3 pointing into the opening 5 and thus into the aligned needle storage compartment 2 in both direction, in particular into or out off the needle storage device 1, the indicator arm 17 is tensioned to its maximum. In this position, there is no sideways guidance on the crank 18 so that the indicator arm 17 jumps upwards and thereby relaxes and thus activates an acoustic as well as a tactile feedback in the area of the touch element 9 in the outer surface of the housing 4.

In other words, after removing of the drug delivery device without a used needle by screwing off the needle connection, e.g. thread or bayonet connection, the indicator 10 gains height opposite to the direction of arrow P2 compared to the housing 4 and the needle storage device 1. In this position in which the needle connection is opened the flexible indicator arm 17 slips over the crank 18 and activates the acoustic and tactile feedback. Now, the user of the device knows that he can remove the drug delivery device from the needle storage device 1.

In an exemplary embodiment the touch element 9 is arranged in the surface of the housing 4 in that area which lies above of the indicator 10, in particular above the indicator arm 17.

During removal of the drug delivery device from the needle storage device 1, the indicator 10 follows this movement until its edge 11.2 is stopped by the fin 12 thereby the spring 13 is relaxed.

Due to the acoustic and/or tactile feedback of the indicator 10 integrated in the needle storage device 1, the user knows if the needle 3 of the drug delivery device is detached in the needle storage compartment 2 which is aligned with the opening 5 of the needle storage device 1. Furthermore, in a case of a needle storage device 1 with a chain of needle storage compartments 2 it is not possible to insert the needle back again into the needle storage compartment after removing the needle because an automatic mechanism is provided that allows to move "used" or "occupied" needle storage compartments.

In an exemplary embodiment the needle storage device 1, 1', 1" and/or the spring 13 may comprise or consist of at least one material with a flexible behaviour (high strained) combined with sufficient strength. Such materials may for example be spring steel sheet or semi-crystalline materials, e.g. Polyamide, Polyoxymethylene, Polybutylene terephthalate, Polyethylene or Polypropylene or a thermoplastic elastomer. Spring steel sheet is particularly suitable for providing the acoustic feedback.

Those of skill in the art will understand that modifications (additions and/or removals) of various components of the apparatuses, and embodiments described herein may be made within the scope defined by the appended claims.

## Claims

1. Needle storage device (1) comprising:
- a housing (4) having an opening (5) adapted to receive a needle (3) of a drug delivery device;
- needle storage compartments (2) moveably disposed within the housing (4);
- an indicator (10) pivotably disposed within the housing (4) and adapted to activate an acoustic and/or tactile feedback when a needle (3) of a drug delivery device is stored in one of the needle storage compartments (2) which is aligned with the opening (5), wherein the indicator (10) comprises a base plate (11) pivotably mounted to the housing (4) about a pivotal axis (D), wherein in the area of the opening (5) a fin (12) is provided and adapted to serve as a stop for the base plate (11) in an initial state in which no drug delivery device is inserted in the opening (5), wherein an indicator arm (17) is provided which is flexible and guided on a crank (18) comprising a ramp formed by an inner surface of the housing (4), wherein after storage of the needle (3) and removal of the drug delivery device without the needle (3) the indicator (10) arm slides over the ramp of the crank (18) and activates the acoustic and/or tactile feedback.

2. Needle storage device (1) according to claim 1, wherein a spring (13) is arranged between the indicator (10) and the housing (4).

3. Needle storage device (1) according to claim 2, wherein the spring (13) is arranged on the pivotal axis (D) of the indicator (10).

4. Needle storage device (1) according to claim 2 or 3, wherein the spring (13) is adapted to be tensioned between the indicator (10) and the housing (4) during insertion of the drug delivery device by pivoting of the indicator (10) with respect to the housing (4).

5. Needle storage device (1) according to one of the preceding claims 2 to 4, wherein the spring (13) is formed as a leg spring.

6. Needle storage device (1) according to one of the preceding claims 1 to 5, wherein the base plate (11) and the housing (4) are moveable relative to each other by a slot-pin guidance (16).

7. Needle storage device (1) according to any one of the preceding claims, wherein the crank (18) comprises at least one ramp on which the indicator arm (17) is guided and prestressed during insertion of the drug delivery device with its needle (3) pointing into the opening (5) as well as into the aligned needle storage compartment (2).

8. Needle storage device (1) according to any of the preceding claims, wherein the housing (4) comprises an outer shape and/or sections with a substantially circular, semicircular, oval or elliptic shape.

9. Needle storage device (1) according to any of the preceding claims, wherein a door (6) is provided allowing access to the opening (5).

10. Needle storage device (1) according to any of the preceding claims, wherein a drive and conveyor chain (8) is provided and adapted to deliver the needle storage compartments (2) within the housing (4) such that at least one of the needle storage compartments (2) is aligned with the opening (5).

## Patentansprüche

1. Nadelaufbewahrungsvorrichtung (1), umfassend:
- ein Gehäuse (4) mit einer Öffnung (5), die zur Aufnahme einer Nadel (3) einer Medikamenten-Verabreichungsvorrichtung ausgelegt ist;
- Nadelaufbewahrungsfächer (2), die beweglich im Gehäuse (4) angeordnet sind;
- eine Anzeige (10), die schwenkbar im Gehäuse (4) angeordnet ist und zur Aktivierung einer akustischen und/oder taktilen Rückmeldung ausgelegt ist, wenn eine Nadel (3) einer Medikamenten-Verabreichungsvorrichtung in einem der Nadelaufbewahrungsfächer (2) aufbewahrt ist, die mit der Öffnung (5) ausgerichtet ist, wobei die Anzeige (10) eine Grundplatte (11) umfasst, die um eine Schwenkachse (D) schwenkbar am Gehäuse (4) angebracht ist, wobei im Bereich der Öffnung (5) eine Finne (12) vorgesehen ist und dazu ausgelegt ist, als Sperre für die Grundplatte (11) in einem Anfangszustand zu dienen, in dem keine Medikamenten-Verabreichungsvorrichtung in die Öffnung (5) eingeführt ist, wobei ein Anzeigearm (17) vorgesehen ist, der flexibel ist und auf einer Kurbel (18) geführt wird, die eine Rampe umfasst, die von einer Innenseite des Gehäuses (4) ausgebildet wird, wobei der Arm der Anzeige (10) nach Aufbewahren der Nadel (3) und Entfernen der Medikamenten-Verabreichungsvorrichtung ohne die Nadel (3) über die Rampe der Kurbel (18) gleitet und die akustische und/oder taktile Rückmeldung aktiviert.

2. Nadelaufbewahrungsvorrichtung (1) nach Anspruch 1, wobei eine Feder (13) zwischen der Anzeige (10) und dem Gehäuse (4) angeordnet ist.

3. Nadelaufbewahrungsvorrichtung (1) nach Anspruch 2, wobei die Feder (13) auf der Schwenkachse (D) der Anzeige (10) angeordnet ist.

4. Nadelaufbewahrungsvorrichtung (1) nach Anspruch 2 oder 3, wobei die Feder (13) dazu ausgelegt ist, zwischen der Anzeige (10) und dem Gehäuse (4) während der Einführung der Medikamenten-Verabreichungsvorrichtung gespannt zu werden, indem die Anzeige (10) bezüglich des Gehäuses (4) geschwenkt wird.

5. Nadelaufbewahrungsvorrichtung (1) nach einem der vorhergehenden Ansprüche 2 bis 4, wobei die Feder (13) als Schenkelfeder ausgebildet ist.

6. Nadelaufbewahrungsvorrichtung (1) nach einem der vorhergehenden Ansprüche 1 bis 5, wobei die Grundplatte (11) und das Gehäuse (4) in Bezug aufeinander durch eine Schlitz-Stift-Führung (16) beweglich sind.

7. Nadelaufbewahrungsvorrichtung (1) nach einem der vorhergehenden Ansprüche, wobei die Kurbel (18) mindestens eine Rampe umfasst, auf der der Hinweisarm (17) geführt wird und während der Einführung der Medikamenten-Verabreichungsvorrichtung vorgespannt wird, wobei ihre Nadel (3) in die Öffnung (5) und in die das ausgerichtete Nadelaufbewahrungsfach (2) zeigt.

8. Nadelaufbewahrungsvorrichtung (1) nach einem der vorhergehenden Ansprüche, wobei das Gehäuse (4) eine Außenform und/oder Abschnitte mit einer im Wesentlichen kreisförmigen, halbkreisförmigen, ovalen oder ellipsenförmigen Gestalt umfasst.

9. Nadelaufbewahrungsvorrichtung (1) nach einem der vorhergehenden Ansprüche, wobei eine Tür (6) vorgesehen ist, die Zugang zur Öffnung (5) erlaubt.

10. Nadelaufbewahrungsvorrichtung (1) nach einem der vorhergehenden Ansprüche, wobei eine Antriebs- und Förderkette (8) vorgesehen und dazu ausgelegt ist, die Nadelaufbewahrungsfächer (2) im Gehäuse (4) zu liefern, so dass mindestens eines der Nadelaufbewahrungsfächer (2) mit der Öffnung (5) ausgerichtet ist.

## Revendications

1. Dispositif (1) de stockage d'aiguilles comprenant :
- un boîtier (4) comportant une ouverture (5) apte à recevoir une aiguille (3) d'un dispositif d'administration de médicaments ;
- des compartiments (2) de stockage d'aiguille disposés mobiles à l'intérieur du boîtier (4) ;
- un indicateur (10) disposé pivotant à l'intérieur du boîtier (4) et apte à activer une rétroaction acoustique et/ou tactile quand une aiguille (3) d'un dispositif d'administration de médicaments est stockée dans un des compartiments (2) de stockage d'aiguille qui est aligné avec l'ouverture (5), dans lequel l'indicateur (10) comprend une plaque de base (11) montée pivotante sur le boîtier (4) autour d'un axe de pivotement (D),
dans lequel une ailette (12) est disposée dans la zone de l'ouverture (5) et apte à servir de butée pour la plaque de base (11) dans un état initial dans lequel aucun dispositif d'administration de médicaments n'est introduit dans l'ouverture (5), dans lequel est utilisé un bras (17) d'indicateur qui est flexible et guidé sur une manivelle (18) comprenant une rampe constituée par une surface intérieure du boîtier (4),
dans lequel, après stockage de l'aiguille (3) et retrait du dispositif d'administration de médicaments sans l'aiguille (3), le bras de l'indicateur (10) glisse sur la rampe de la manivelle (18) et active la rétroaction acoustique et/ou tactile.

2. Dispositif (1) de stockage d'aiguilles selon la revendication 1, dans lequel un ressort (13) est disposé entre l'indicateur (10) et le boîtier (4).

3. Dispositif (1) de stockage d'aiguilles selon la revendication 2, dans lequel le ressort (13) est disposé sur l'axe de pivotement (D) de l'indicateur (10).

4. Dispositif (1) de stockage d'aiguilles selon la revendication 2 ou 3, dans lequel le ressort (13) est apte à être tendu entre l'indicateur (10) et le boîtier (4) pendant l'introduction du dispositif d'administration de médicaments en faisant pivoter l'indicateur (10) par rapport au boîtier (4).

5. Dispositif (1) de stockage d'aiguilles selon l'une quelconque des revendications 2 à 4, dans lequel le ressort (13) se présente sous la forme d'un ressort de maintien.

6. Dispositif (1) de stockage d'aiguilles selon l'une quelconque des revendications 1 à 5, dans lequel la plaque de base (11) et le boîtier (4) sont mobiles l'un par rapport à l'autre grâce à un guidage (16) fente-clavette.

7. Dispositif (1) de stockage d'aiguilles selon l'une quelconque des revendications précédentes, dans lequel la manivelle (18) comprend au moins une rampe sur laquelle le bras (17) d'indicateur est guidé et précontraint pendant l'introduction du dispositif d'administration de médicaments avec son aiguille (3) tournée vers l'ouverture (5) ainsi que vers le compartiment (2) de stockage d'aiguille aligné.

8. Dispositif (1) de stockage d'aiguilles selon l'une quelconque des revendications précédentes, dans lequel le boîtier (4) présente une forme extérieure et/ou des sections ayant une forme sensiblement circulaire, semi-circulaire, ovale ou elliptique.

9. Dispositif (1) de stockage d'aiguilles selon l'une quelconque des revendications précédentes, dans lequel on trouve une porte (6) qui permet l'accès à l'ouverture (5).

10. Dispositif (1) de stockage d'aiguilles selon l'une quelconque des revendications précédentes, dans lequel une chaîne (8) d'entraînement et de transport est utilisée et apte à distribuer les compartiments (2) de stockage d'aiguille à l'intérieur du boîtier (4) de telle sorte qu'au moins un des compartiments (2) de stockage d'aiguille soit aligné avec l'ouverture (5).
